(19) Europäisches Patentamt
European Patent Office
Office européen des brevets

(11) Numéro de publication : **0 332 521 B1**

(12) # FASCICULE DE BREVET EUROPEEN

(45) Date de publication du fascicule du brevet : **07.10.92 Bulletin 92/41**

(51) Int. Cl.$^5$ : **C07D 327/10**

(21) Numéro de dépôt : **89400619.6**

(22) Date de dépôt : **06.03.89**

(54) **Procédé de préparation de sulfates cycliques.**

Le dossier contient des informations techniques présentées postérieurement au dépôt de la demande et ne figurant pas dans le présent fascicule.

(30) Priorité : **08.03.88 FR 8802917**

(43) Date de publication de la demande : **13.09.89 Bulletin 89/37**

(45) Mention de la délivrance du brevet : **07.10.92 Bulletin 92/41**

(84) Etats contractants désignés : **AT BE CH DE ES FR GB GR IT LI LU NL SE**

(56) Documents cités :
**EP-A- 399 899**
**CHEMICAL ABSTRACTS, vol. 108, 1988, page 601, résumé no. 37493n, Columbus, Ohio, US**

(56) Documents cités :
**JOURNAL OF CHEMICAL SOCIETY, CHEM. COMM., 1983, pages 1392-1394, Londres, GB; G. LOWE et al.: "Application of a lanthanideshift reagent in 170 N.M.R. spectroscopy to determine the stereochemical course of oxidation of cyclic sulphite diesters to cyclicsulphate diesters with ruthenium tetroxide"**

(73) Titulaire : **RHONE-POULENC SANTE**
**20, avenue Raymond Aron**
**F-92160 Antony (FR)**

(72) Inventeur : **Le Roy, Pierre**
**16 Avenue de Grande-Bretangne**
**F-69006 Lyon (FR)**
Inventeur : **Mandard-Cazin, Bernadette**
**32 rue des Ecoles**
**F-94140 Alfortville (FR)**

(74) Mandataire : **Pilard, Jacques et al**
**RHONE-POULENC RORER S.A. Direction Brevets (t144) 20 avenue Raymond Aron**
**F-92165 Antony Cédex (FR)**

**EP 0 332 521 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne un procédé de préparation de sulfates cycliques par oxydation des sulfites cycliques correspondants.

Il est connu, en particulier de l'article de G. Lowe et S.J. Salamone, J. Chem. Soc., Chem. Comm., 1392-1394 (1983), que les sulfates cycliques de formule générale (I) peuvent être obtenus par oxydation des sulfites cycliques de formule générale (II) au moyen de tétroxyde de ruthénium préparé in situ à partir d'oxyde de ruthénium ($RuO_2$) et de périodate de sodium. Cependant la mise en oeuvre de ce procédé nécessite l'emploi de l'oxyde de ruthénium (IV) en quantité stoechiométrique et du périodate de sodium qui sont des produits coûteux.

D'après le brevet espagnol ES 533 294 [Chem. Abstr., 108, 37493 (1988)], il est connu d'oxyder des sulfures en sulfoxydes et sulfones au moyen d'hypochlorite ou de periodate de sodium en présence d'une quantité catalytique de sel de ruthénium.

La présente invention a pour objet un procédé de préparation de sulfates cycliques de formule générale :

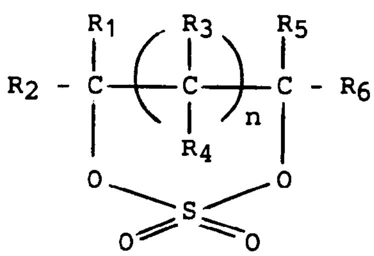

(I)

dans laquelle les substituants $R_1$, $R_2$, $R_3$, $R_4$, $R_5$ et $R_6$, identiques ou différents, représentent un atome d'hydrogène ou d'halogène, un radical alkyle, aryle, alkoxy, aryloxy, ou alkoxycarbonyle et n est égal à 0 ou 1, étant entendu que les radicaux alkyles et les portions alkyles des radicaux alkoxy ou alkoxycarbonyles contiennent 1 à 4 atomes de carbone et peuvent être éventuellement substitués par un ou plusieurs atomes ou radicaux identiques ou différents choisis parmi les atomes d'halogène ou radicaux alkoxy, aryloxy ou alkoxycarbonyles et que les radicaux aryles et les portions aryles des radicaux aryloxy contiennent 6 à 10 atomes de carbone et peuvent être éventuellement substitués par un ou plusieurs atomes ou radicaux identiques ou différents choisis parmi les atomes d'halogène ou radicaux alkyles, alkoxy, aryloxy ou alkoxycarbonyles par oxydation d'un sulfite cyclique de formule générale :

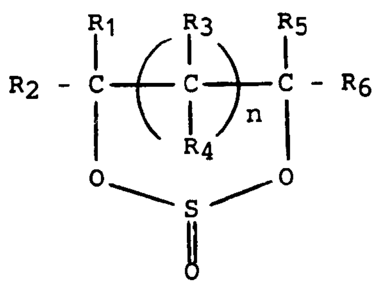

(II)

dans laquelle $R_1$, $R_2$, $R_3$, $R_4$, $R_5$, $R_5$ et n sont définis comme précédemment au moyen d'un hypohalogénite alcalin ou alcalino-terreux, en présence d'une quantité catalytique d'un dérivé du ruthénium en milieu aqueux ou hydro-organique à une température inférieure à 10°C.

L'hypohalogénite alcalin ou alcalino-terreux est choisi parmi les hypochlorite ou hypobromite de sodium, de potassium ou de calcium.

Le dérivé du ruthénium est choisi de préférence parmi l'oxyde de ruthénium (IV)($RuO_2$) et le chlorure de ruthénium ($RUCl_3$).

Le procédé selon la présente invention peut être mis en oeuvre en milieu aqueux ou hydroorganique biphasique.

Lorsque le procédé est mis en oeuvre en milieu hydroorganique biphasique le solvant est généralement choisi parmi les hydrocarbures aliphatiques ou cycloaliphatiques éventuellement halogénés, tels que l'hexane,

le cyclohexane, le chlorure de méthylène, le chloroforme, le tétrachlorure de carbone ou le dichloroéthane, ou les esters tels que l'acétate de méthyle ou l'acétate d'éthyle.

Généralement, on utilise un excès d'hypohalogénite par rapport au sulfite de formule générale (II) mis en oeuvre. De préférence on utilise 1 à 2 moles d'hypohalogénite par mole de sulfite de formule générale (II).

Généralement, on utilise une quantité catalytique de dérivé du ruthénium comprise entre $10^{-6}$ et $10^{-1}$ mole par mole de sulfite de formule générale (II).

Le sulfate cyclique de formule générale (I) est séparé du mélange réactionnel selon les techniques habituelles.

Le sulfite de formule générale (II) peut être obtenu selon les méthodes connues et en particulier selon le procédé décrit par D.S. Breslow et H. Skolnik, "The Chemistry of Heterocyclic Compounds - Multi-Sulphur and Sulphur and Oxygen 5- and 6- Membered Heterocycles, 1966, Part I, p. 1 et Part II, p. 663 ou par H.F. Van Woerden, Chem. Rev., 63, 557 (1963).

Plus particulièrement, la présente invention concerne la préparation des sulfates cycliques de formule générale (I) dans laquelle n est égal à 0 ou 1 et les symboles $R_1$, $R_2$, $R_3$, $R_4$, $R_5$ et $R_6$, identiques ou différents, représentent un atome d'hydrogène ou un radical alkyle.

Les produits de formule générale (I) sont des intermédiaires utilisables en chimie organique pour réaliser en particulier des réactions d'hydroxyalkylation.

Les exemples suivants, donnés à titre non limitatif, montrent comment l'invention peut être mise en pratique.

## EXEMPLE 1

Dans un ballon de 250 cm3, on introduit 27 g de sulfite d'éthylène (0,25 mole), 175 cm3 de dichlorométhane et 18 mg d'oxyde de ruthénium (IV), dihydrate (0,106 x $10^{-3}$ mole). On refroidit à 5°C puis on ajoute, en 40 minutes, sous forte agitation, 125 cm3 d'une solution d'hypochlorite de sodium à 2 moles par litre (0,25 mole).

On poursuit l'agitation pendant 10 minutes à 5°C.

Après décantation, la phase organique est agitée avec 1 cm3 d'isopropanol pendant 10 minutes à 10°C puis elle est lavée par 40 cm3 d'eau à 5°C.

Après concentration de la phase organique à 30°C sous pression réduite (100 mm de mercure ; 13,3 kPa), on obtient 25,4 g de sulfate d'éthylène (0,205 mole) sous forme de cristaux blancs fondant à 99°C.

Le rendement est de 82 %.

## EXEMPLE 2

On opère comme dans l'exemple 1, en remplaçant le dichlorométhane par de l'acétate de méthyle.

On obtient ainsi 24,3 g de sulfate d'éthylène sous forme de cristaux blancs fondant à 99°C.

Le rendement est de 78,2 %.

## EXEMPLE 3

Dans un ballon de 250 cm3, on introduit 13,5 g de sulfite d'éthylène (0,125 mole) et 108 cm3 d'eau. On refroidit à 5°C puis on ajoute en 30 minutes, sous forte agitation, un mélange de 62 cm3 de solution d'hypochlorite de sodium à 2 moles par litre (0,137 mole) et de 9 mg d'oxyde de ruthénium (IV) dihydrate (0,053.$10^{-3}$ mole). On poursuit l'agitation pendant 10 minutes à 5°C. Le précipité obtenu est séparé par filtration puis est lavé par 40 cm3 d'eau à 5°C.

Après séchage à 20°C pendant 17 heures sous pression réduite (10 mm de mercure ; 1,3 kPa), on recueille 10,4 g de sulfate d'éthylène sous forme de cristaux blancs fondant à 99°C.

Le rendement est de 67 %.

## EXEMPLE 4

On opère comme dans l'exemple 3 en remplaçant l'oxyde de ruthénium (IV) dihydrate par 13,1 mg de chlorure de ruthénium trihydrate ($RuCl_3$, $3H_20$), (0,05 x $10^{-3}$ mole).

On obtient ainsi 10,6 g de sulfate d'éthylène sous forme de cristaux blancs fondant à 99°C.

Le rendement est de 68,4 %.

EXEMPLE 5

Dans un ballon de 1 litre, on introduit, sous agitation, 250 cm3 de dichlorométhane, 50 cm3 d'une solution d'hypochlorite de sodium à 2,18 moles par litre et 0,15 g de chlorure de ruthénium trihydrate ($RuCl_3$, $3H_20$), ($5,7.10^{-4}$ mole). On refroidit à 5°C puis on ajoute simultanément, en 40 minutes à 5°C, 73,2 g de sulfite de propylène (0,6 mole) et 300 cm3 d'une solution d'hypochlorite de sodium à 2,18 moles par litre. On poursuit l'agitation pendant 25 minutes à 5°C puis on ajoute 2 cm3 d'isopropanol et agite pendant 20 minutes.

Après décantation, la phase aqueuse est extraite par 2 fois 100 cm3 de dichlorométhane. Les phases organiques réunies sont lavées par 200 cm3 d'eau à 5°C puis séchées sur sulfate de magnésium. Après filtration et évaporation du solvant sous pression réduite (20 mm de mercure ; 2,6 kPa) on obtient 72 g de sulfate de propylène sous forme d'une huile jaune clair dont le point d'ébullition est de 80°C sous 1 mm de mercure (0,13 kPa).

Le rendement est de 87 %.

EXEMPLE 6

Dans un ballon muni d'un agitateur, on introduit 9,00 g de sulfite de diméthyl-1,2 éthylène (0,066 mole), 90 cm3 de dichlorométhane et $7 \times 10^{-3}$ g d'oxyde de ruthénium dihydrate ($RuO_2$, $2H_2O$) ($0,041 \times 10^{-3}$ mole). Le mélange est refroidi à 5°C, puis on ajoute en 40 minutes, sous forte agitation, 35 cm3 d'une solution d'hypochlorite de sodium à 2,1 moles/litre (0,073 mole).

L'agitation du mélange biphasique est maintenue pendant 10 minutes à 5°C. Après décantation, la phase organique est agitée avec 0,5 cm3 d'isopropanol pendant 10 minutes à 5°C, puis lavée avec 50 cm3 d'eau glacée.

Après concentration de la phase organique à 40°C sous pression réduite (100 mm de mercure ; 13,3 kPa) on obtient 7,0 g d'une huile dont le dosage par le spectre infra-rouge montre qu'elle est constituée de sulfate de diméthyl-1,2 éthylène (80 %) et de sulfite de diméthyl-1,2 éthylène (20 %) sous forme d'huile.

Le rendement est de 57 %.

EXEMPLE 7

Dans un ballon muni d'un agitateur, on introduit 8,00 g de sulfite de chlorométhyl-1 éthylène (0,051 mole), 80 cm3 de dichlorométhane et 10 mg de chlorure de ruthénium trihydrate ($0,38.10^{-4}$ mole). On refroidit le mélange à 5°C, puis on coule en 40 minutes 25 cm3 d'une solution d'hypochlorite de sodium à 2,2 moles/litre (0,055 mole). On poursuit l'agitation pendant 10 minutes à 5°C.

Après décantation, on ajoute 2 cm3 d'isopropanol à la phase organique, puis on agite pendant 10 minutes à 5°C. On lave 3 fois avec 50 cm3 d'eau. Après concentration de la phase organique à 40°C sous pression réduite (100 mm de mercure ; 13,3 kPa) on obtient 2,40 g d'une huile dont le dosage par le spectre infra-rouge montre qu'elle contient 27 % de sulfate de chlorométhyl-1 éthylène.

Le rendement est de 8 %.

EXEMPLE 8

Dans un ballon muni d'un agitateur, on introduit 8,00 g de sulfite de diméthyl-2,2 propylène (0,053 mole), 50 cm3 de dichlorométhane et 9,3 mg d'oxyde de ruthénium dihydrate ($RuO_2$, $2H_2O$). On refroidit le mélange à 5°C, puis on ajoute en 40 minutes 24 cm3 d'une solution d'hypochlorite de sodium à 2,2 moles/litre. On maintient l'agitation pendant 20 minutes à 5°C.

Après décantation, on ajoute 2 cm3 d'isopropanol à la phase organique et agite pendant 10 minutes à 5°C. On lave avec 50 cm3 d'eau, décante et sèche la phase organique sur sulfate de sodium. Après concentration à 45°C sous pression réduite (100 mm de mercure ; 13,3 kPa) on obtient 3,9 g d'un mélange dont le dosage par le spectre infra-rouge montre qu'il est constitué de sulfate de diméthyl-2,2 propylène (55 %) et de sulfite de diméthyl-2,2 propylène (45 %).

Le rendement est de 24 %.

EXEMPLE 9

Dans un ballon muni d'un agitateur, on introduit 8,00 g de sulfite de diméthyl-2,2 propylène (0,053 mole), 40 cm3 d'eau et 9,3 mg d'oxyde de ruthénium dihydrate ($RuO_2$, $2H_2O$). On refroidit le mélange à 5°C, puis on ajoute en 60 minutes 24 cm3 d'une solution d'hypochlorite de sodium à 2,2 moles/litre. On maintient l'agitation

pendant 1 heure 30 minutes à 5°C. Le précipité obtenu est séparé par filtration, puis lavé à l'eau glacée.

Après séchage à 20°C pendant 12 heures sous pression réduite (10 mm de mercure ; 1,33 kPa), on récupère 5,3 g de sulfate de diméthyl-2,2 propylène fondant à 77°C.

Le rendement est de 60 %.

Analyse élémentaire :

| | | | | | | | | |
|---|---|---|---|---|---|---|---|---|
| % calculé | C | 36,15 | H | 6,02 | O | 38,53 | S | 19,30 |
| % trouvé | | 35,76 | | 6,32 | | 38,66 | | 19,05 |

EXEMPLE 10

Dans un ballon muni d'un agitateur, on introduit 2,1 g de sulfite de tétraméthyléhylène, 20 cm3 d'eau et 2,5 mg d'oxyde de ruthénium dihydrate. On refroidit le mélange à 15°C et on ajoute en 45 minutes 6,4 cm3 de solution d'hypochlorite de sodium à 2,2 moles/ litre. On maintient l'agitation pendant 1 heure à 15°C. Le précipité est récupéré par filtration, puis lavé avec 10 cm3 d'eau à 15°C.

Après séchage pendant 18 heures à 20°C sous pression réduite (10 mm de mercure ; 1,33 kPa) on obtient 0,6 g de sulfate de tétraméthyléthylène.

Le rendement est de 24 %.

Analyse élémentaire :

| | | | | | | | | |
|---|---|---|---|---|---|---|---|---|
| % calculé | C | 40,01 | H | 6,60 | O | 35,53 | S | 17,80 |
| % trouvé | | 38,90 | | 6,70 | | 35,65 | | 17,56 |

## Revendications

1 - Procédé de préparation d'un sulfate cyclique de formule générale :

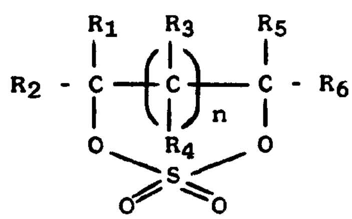

dans laquelle $R_1$, $R_2$, $R_3$, $R_4$, $R_5$ et $R_6$, identiques ou différents, représentent un atome d'hydrogène ou d'halogène ou un radical alkyle, aryle, alkoxy, aryloxy ou alkoxycarbonyle et n est égal à 0 ou 1, étant entendu que les radicaux alkyles et les portions alkyles des radicaux alkoxy ou alkoxycarbonyles contiennent 1 à 4 atomes de carbone et sont éventuellement substitués par un ou plusieurs atomes ou radicaux, identiques ou différents choisis parmi les atomes d'halogène ou radicaux alkoxy, aryloxy ou alkoxycarbonyles, et que les radicaux aryles et les portions aryles des radicaux aryloxy contiennent 6 à 10 atomes de carbone et sont éventuellement substitués par un ou plusieurs atomes ou radicaux, identiques ou différents, choisis parmi les atomes d'halogène, les radicaux alkyles, alkoxy, aryloxy ou alkoxycarbonyles, par oxydation d'un sulfite cyclique de formule générale :

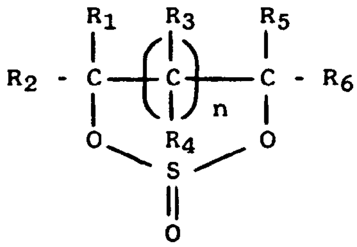

dans laquelle $R_1$, $R_2$, $R_3$, $R_4$, $R_5$, $R_5$ et n sont définis comme précédemment, caractérisé en ce que l'oxydation est réalisée au moyen d'un hypohalogénite alcalin ou alcalino-terreux en présence d'une quantité d'un dérivé de ruthénium à raison de $10^{-6}$ à $10^{-1}$ mole par mole de sulfite cyclique en opérant en milieu aqueux ou hydroorganique à une température inférieure à 10°C.

2 - Procédé selon la revendication 1 caractérisé en ce que le dérivé du ruthénium est choisi parmi l'oxyde de ruthénium (IV) et le trichlorure de ruthénium.

3 - Procédé selon la revendication 1 caractérisé en ce que l'hypohalogénite alcalin ou alcalino-terreux est choisi parmi l'hypochlorite de sodium l'hypochlorite de potassium et l'hypochlorite de calcium.

4 - Procédé selon l'une des revendications 1 ou 3 caractérisé en ce que l'on utilise 1 à 2 moles d'hypohalogénite par mole de sulfite cyclique.

5 - Procédé selon la revendication 1 caractérisé en ce que, lorsque l'on opère en milieu hydro-organique, le solvant est choisi parmi les hydrocarbures aliphatiques ou cycloaliphatiques éventuellement halogénés et les esters.

6 - Procédé selon l'une des revendications 1 à 5 pour la préparation d'un sulfate cyclique de formule générale :

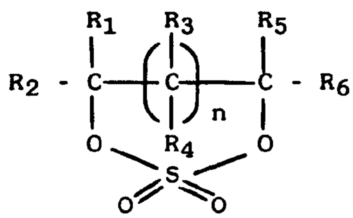

dans laquelle $R_1$, $R_2$, $R_3$, $R_4$, $R_5$ et $R_5$, identiques ou différents, représentent un atome d'hydrogène ou un radical alkyle contenant 1 à 4 atomes de carbone et n est égal à 0 ou 1.

7 - Procédé selon l'une des revendications 1 à 5 pour la préparation du sulfate d'éthylène ou du sulfate de propylène.

8 - Le sulfate de chlorométhyl-1 éthylène.

**Patentansprüche**

1. Verfahren zur Herstellung eines cyclischen Sulfats der allgemeinen Formel:

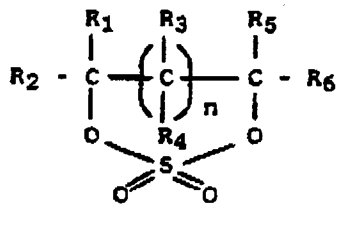

in welcher $R_1$, $R_2$, $R_3$, $R_4$, $R_5$ und $R_6$, die gleich oder voneinander verschieden sind, ein Wasserstoff- oder Halogenatom oder einen Alkyl-, Aryl-, Alkoxy-, Aryloxy- oder Alkoxycarbonylrest darstellen und n gleich 0 oder 1 ist, wobei die Alkylreste und die Alkylteile der Alkoxy- oder Alkoxycarbonylreste selbstverständlich

1 bis 4 Kohlenstoffatome enthalten und gegebenenfalls durch ein oder mehrere gleiche oder voneinander verschiedene Atome oder Reste, ausgewählt aus den Halogenatomen oder Alkoxy- Aryloxy- oder Alkoxycarbonylresten, substituiert sind, und die Arylreste und die Arylteile der Aryloxyreste 6 bis 10 Kohlenstoffatome enthalten und gegebenenfalls durch ein oder mehrere gleiche oder voneinander verschiedene Atome oder Reste, ausgewählt aus den Halogenatomen, den Alkyl-, Alkoxy-, Aryloxy- oder Alkoxycarbonylresten, substituiert sind, durch Oxidation eines cyclischen Sulfits der allgemeinen Formel:

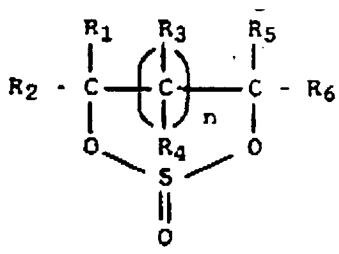

in welcher $R_1$, $R_2$, $R_3$, $R_4$, $R_5$ und $R_6$ und n die obige Bedeutung haben, dadurch gekennzeichnet, daß die Oxidation mittels eines Alkali- oder Erdalkalihypohalogenits in Gegenwart einer Menge einer Rutheniumverbindung im Anteil von $10^{-6}$ bis $10^{-1}$ mol pro mol cyclisches Sulfit ausgeführt wird, indem man in wasserigem oder wässerig-organischem Milieu bei einer Temperatur unterhalb 10°C arbeitet.

**2.** Verfahren nach Anspruch 1, dadurch gekennzeichnet, daß die Rutheniumverbindung ausgewählt ist aus Ruthenium(IV)oxid und Rutheniumtrichlorid.

**3.** Verfahren nach Anspruch 1, dadurch gekennzeichnet, daß das Alkali- oder Erdalkalihypohalogenit ausgewählt ist aus Natriumhypochlorit, Kaliumhypochlorit und Calciumhypochlorit.

**4.** Verfahren nach einem der Ansprüche 1 oder 3, dadurch gekennzeichnet, daß man 1 bis 2 mol Hypohalogenit pro mol cyclisches Sulfit verwendet.

**5.** Verfahren nach Anspruch 1, dadurch gekennzeichnet, daß das Lösungsmittel, wenn man in wässerig-organischem Milieu arbeitet, ausgewählt ist aus den aliphatischen oder cycloaliphatischen, gegebenenfalls halogenierten Kohlenwasserstoffen und den Estern.

**6.** Verfahren nach einem der Ansprüche 1 bis 5 zur Herstellung eines cyclischen Sulfats der allgemeinen Formel:

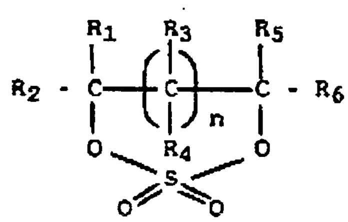

in welcher $R_1$, $R_2$, $R_3$, $R_4$, $R_6$ und $R_6$ unabhängig voneinander ein Wasserstoffatom oder einen Alkylrest mit 1 bis 4 Kohlenstoffatomen darstellen und n gleich 0 oder 1 ist.

**7.** Verfahren nach einem der Ansprüche 1 bis 5 zur Herstellung von Äthylensulfat oder Propylensulfat.

**8.** 1-Chlormethyläthylensulfat.

## Claims

1 - A process for the preparation of a cyclic sulphate of general formula:

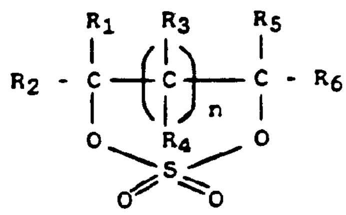

in which $R_1$, $R_2$, $R_3$, $R_4$, $R_5$ and $R_6$ are identical or different and represent a hydrogen or halogen atom or an alkyl, aryl, alkoxy, aryloxy or alkoxycarbonyl radical and n is equal to 0 or 1, it being understood that the alkyl radicals and alkyl portions of the alkoxy or alkoxycarbonyl radicals contain 1 to 4 carbon atoms and may optionally be substituted by one or more identical or different atoms or radicals selected among halogen atoms or alkoxy, aryloxy or alkoxycarbonyl radicals, and that the aryl radicals and the aryl portions of the aryloxy radicals contain 6 to 10 carbon atoms and are optionally substituted by one or more identical or different atoms or radicals selected among halogen atoms or alkyl, alkoxy, aryloxy or alkoxycarbonyl radicals, by oxidation of a cyclic sulphite of general formula:

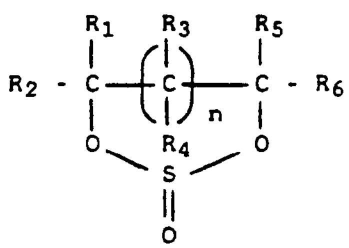

in which $R_1$, $R_2$, $R_3$, $R_4$, $R_5$, $R_6$ and n are as previously defined, wherein the oxidation is carried out by means of a hypohalite of an alkali or alkaline-earth metal in the presence of a quantity of a ruthenium derivative in the proportion of from $10^{-6}$ to $10^{-1}$ mole per mole of cyclic sulphite, operating in an aqueous or aqueous-organic medium at a temperature below 10°C.

2 - A process according to claim 1 in which the ruthenium derivative is selected among ruthenium (IV) oxide and ruthenium trichloride.

3 - A process according to claim 1 in which the hypohalite of an alkali or alkaline-earth metal is selected among sodium hypochlorite, potassium hypochlorite and calcium hypochlorite.

4 - A process according to one of claims 1 or 3 in which 1 to 2 moles of hypohalite are used per mole of cyclic sulphite.

5 - A process according to claim 1 in which, when operating in an aqueous-organic medium, the solvent is selected among the optionally halogenated aliphatic or cycloaliphatic hydrocarbons and esters.

6 - A process according to one of claims 1 to 5 for the preparation of a cyclic sulphate of general formula:

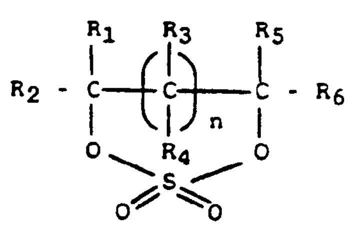

in which $R_1$, $R_2$, $R_3$, $R_4$, $R_6$ and $R_6$ are identical or different and represent a hydrogen atom or an alkyl radical containing 1 to 4 carbon atoms and n is equal to 0 or 1.

7 - A process according to one of claims 1 to 5 for the preparation of ethylene sulphate or propylene sulphate.

8 - 1-(Chloromethyl)ethylene sulphate.